(19) **Europäisches Patentamt**

**European Patent Office**

**Office européen des brevets**

(11) **EP 0 777 669 B1**

(12) **EUROPEAN PATENT SPECIFICATION**

(45) Date of publication and mention
of the grant of the patent:
**03.07.2002 Bulletin 2002/27**

(21) Application number: **95929954.6**

(22) Date of filing: **25.08.1995**

(51) Int Cl.⁷: $C07D\ 473/00$, $A61K\ 31/52$

(86) International application number:
**PCT/GB95/02014**

(87) International publication number:
**WO 96/06844 (07.03.1996 Gazette 1996/11)**

(54) **4-(2-AMINO-6-(CYCLOPROPYLAMINO)-9H-PURIN-9-YL)-2-CYCLOPENTENE-1-METHANOL SUCCINATE AS ANTIVIRAL AGENT**

4-[2-AMINO-6-(CYCLOPROPYLAMINO)-9H-PYRIN-9-YL]-2-CYCLOPENTEN-1-METHANOL-SUCCINAT ALS ANTIVIRALES MITTEL

UTILISATION DU SUCCINATE DE 4-(2-AMINO-6-(CYCLOPROPYLAMINO)-9H-PURIN-9-YL)-2-CYCLOPENTENE-METHANOL COMME AGENT ANTIVIRAL

(84) Designated Contracting States:
**AT BE CH DE DK ES FR GB GR IE IT LI LU MC NL PT SE**
Designated Extension States:
**LT LV SI**

(30) Priority: **26.08.1994 GB 9417249**

(43) Date of publication of application:
**11.06.1997 Bulletin 1997/24**

(73) Proprietor: **THE WELLCOME FOUNDATION LIMITED**
**Greenford, Middlesex UB6 0NN (GB)**

(72) Inventors:
• **DALUGE, Susan, Mary**
**Chapel Hill, NC 27514 (US)**

• **WILSON, Jeffrey, Douglas**
**Durham, NC 27705 (US)**

(74) Representative: **Quillin, Helen Kaye et al**
**GlaxoSmithKline**
**Corporate Intellectual Property (CN9.25.1)**
**980 Great West Road**
**Brentford, Middlesex TW8 9GS (GB)**

(56) References cited:
**EP-A- 0 349 242        EP-A- 0 434 450**
**WO-A-94/15614**

Remarks:
The file contains technical information submitted after the application was filed and not included in this specification

**Description**

The present invention relates to a novel salt of (1S,4R)-<u>cis</u>-4-[2-amino-6-(cyclopropylamino)-9H-purin-9-yl]-2-cyclopentene-1-methanol or a solvate thereof, pharmaceutical formulations containing such a compound, to its, or a pharmaceutical formulation, use in medical therapy or methods of treatment or prophylaxis of viral diseases specifically against human immunodeficiency virus (HIV) and hepatitis B virus (HBV) infection.

The compound (1S,4R)-<u>cis</u>-4-[2-amino-6-(cyclopropylamino)-9H-purin-9-yl]-2-cyclopentene-1-methanol and its antiviral use, especially against HIV infections, is described in European patent Specification Number 0434450 which also refers to pharmaceutically acceptable derivatives specifically salts, esters and salts of such esters of the compound, and in particular describes hydrochloride salts of the compound.

The use of a salt of a compound is well known in the art and acid addition salts of the above compound are described in EP0434450. It is difficult to predict the physical characteristics of any particular salt of a compound and small, but significant, differences in physical properties may equate to large savings in the manufacture and formulation of a pharmaceutical product containing that compound.

The compound (1S,4R)-<u>cis</u>-4-[2-amino-6-(cyclopropylamino)-9H-purin-9-yl]-2-cyclopentene-1-methanol is currently under clinical investigation as an anti-HIV pharmaceutical agent. There exists a need for the compound to be prepared in a form suitable for ease of isolation in large scale manufacture, and for ease of formulating into an acceptable product for administration to humans. We have found that manufacture of the free base of the compound produces a gum which traps solvents and is, therefore, unsuitable for large scale purification, or for formulation, without additional purification procedures.

It is an object of the present invention to provide an acid addition salt of (1S,4R)-<u>cis</u>-4-[2-amino-6-(cyclopropylamino)-9H-purin-9-yl]-2-cyclopentene-1-methanol or a solvate thereof which is easily, and reproducibly, prepared from equimolar amounts of the acid and the parent compound on a large scale, with high yields, and where the salt prepared is stable, easy to isolate, for example by rapid and efficient filtration, and phamaceutically acceptable.

We have found that the advantages of the succinate salt of the above compound over the hydrochloride salts disclosed renders the succinate salt particularly suitable and advantageous to prepare on a large scale, and for use in the preparation of pharmaceutical formulations for administration to humans.

According to the first aspect of the invention there is provided the succinate salt of (1S,4R)-<u>cis</u>-4-[2-amino-6-(cyclopropylamino)-9H-purin-9-yl]-2-cyclopentene-1-methanol or a solvate thereof, including a hydrate thereof, hereinafter referred to as the compound according to the invention.

Further aspects of the invention include:

a) The compound of the invention for use in medical therapy, particularly in the treatment or prophylaxis of viral infections, specifically against an HIV or an HBV infection.

b) A method for the treatment or prophylaxis of a viral infection particularly an HIV or an HBV infection in a human which comprises administering to said human an effective amount of the compound according to the invention.

c) Use of a compound according to the invention in the manufacture of a medicament for the treatment or prophylaxis of a viral infection particularly an HIV or an HBV infection.

The compound of the invention is particularly useful for the prophylaxis or treatment of HIV infections.

Examples of clinical conditions caused by HIV infections which may be treated in accordance with the invention include Acquired Immune Deficiency Syndrome (AIDS) or symptoms that frequently precede AIDS, or related clinical conditions such as AIDS-related complex (ARC), progressive generalised lymphadenopathy (PGL), Kaposis sarcoma, thrombocytopenic purpura, AIDS related neurological conditions, such as multiple sclerosis or tropical paraparesis and also anti-HIV antibody-positive and HIV-positive conditions including AIDS asymptomatic patients.

As an additional feature of the invention we provide a process for the preparation of the compound of the invention which comprises dissolving (1S,4R)-<u>cis</u>-4-[2-amino-6-cyclopropylamino)-9H-purin-9-yl]-2-cyclopentene-1-methanol and succinic acid in water, preferably in equimolar amounts, in a suitable solvent, for example aqueous ethanol or isopropanoL Such solutions are advantageously prepared at reflux . Upon cooling to ambient temprature crystals of the compound of the invention precipitate from said solution in high yield. Optional washing and recrystallisation may be used to increase the purity of the product, if required. The compound of the invention being in a crystalline form provides a means for large scale manufacture by rapid and efficient filtration.

The compound of the invention may be administered alone or in combination with other therapeutic agents suitable in the treatment of HIV infections, such as Nucleoside Reverse Transcriptase Inhibitors (NRTIs) for example zidovudine, zalcitabine, lamivudine, didanosine, stavudine, 5-chloro-2',3'-dideoxy-3'-fluorouridine and (2R,5S)-5-fluoro-1-[2-(hydroxymethyl)-1,3-oxathiolan-5-yl]cytosine, non-NRTIs for example nevirapine and α-APA, HIV protease inhibitors for example saquinavir or VX-478, other anti-HIV agents for example soluble CD4, immune modulators for example interleukin II, erythyropoetin, tucaresol, and interferons for example *a*-interferon. In addition the compound of the invention may be administered in combination with other therapeutic agents suitable in the treatment of HBV infections for example lamivudine, (2R,5S)-5-fluoro-1-[2-(hydroxymethyl)-1,3-oxathiolan-5-yl]cytosine, immune modulators, and interferons as described above. Such combinations may be administered together or sequentially providing that any duration between the administration of each therapeutic agent does not diminish their additive effect.

While it is possible for the compound of the invention to be administered alone it is preferable and advantageous to present the compound of the invention as a pharmaceutical formulation, and represents a further feature of the invention. The pharmaceutical formulation comprises of the compound of the invention together with one or more acceptable carrier(s) thereto and optionally other therapeutic agents. The carrier(s) must be "acceptable" in the sense of being compatible with the other ingredients of the formulation and not deleterious to the recipients thereof

The compounds according to the invention may be administered by any route appropriate to the condition to be treated, suitable routes including oral, rectal, nasal, topical (including transdermal, buccal and sublingual), vaginal and parenteral (including subcutaneous, intramuscular, intravenous, intradermal, intrathecal and epidural). It will be appreciated that the preferred route may vary with, for example, the condition of the recipient.

For each of the above-indicated utilities and indications the amount required of the individual active ingredient will depend upon a number of factors including the seventy of the condition to be treated and the identity of the recipient and will ultimately be at the discretion of the attendant physician. In general, however, for each of these utilities and indications, a suitable, effective dose will be in the range of 3 to 120 mg per kilogram body weight of recipient per day, preferably in the range of 6 to 90 mg per kilogram body weight per day and most preferably in the range of 15 to 60 mg per kilogram body weight per day. The dose may if desired be presented as two, three, four or more sub-doses administered at appropriate intervals throughout the day.

The formulations include those suitable for oral, rectal, nasal, topical (including buccal and sublingual), vaginal or parenteral (including subcutaneous, intramuscular, intravenous, intradermal, intrathecal and epidural) administration. The formulations may conveniently be presented in unit dosage form and may be prepared by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, or paste or may be contained within liposomes.

A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g. povidone, gelatin, hydroxypropylmethyl cellulose), lubricant, inert diluent, disintegrant (e.g. sodium starch glycollate, cross-linked povidone, cross-linked sodium carboxymethyl cellulose), surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethyl-cellulose in varying proportions to provide the desired release profile.

A capsule may be made by filling a loose or compressed powder or an appropriate filling machine, optionally with one or more additives. Examples of suitable additives include binders such as povidone, gelatin, lubricants, inert diluents, disintegrants as for tablets. Capsules may also be formulated to contain pellets or discrete sub-units to provide slow or controlled release of the outline ingredient. This can be achieved by extruding and spheronising a wet mixture of the drug plus an extrusion acid (e.g. microcrystalline cellulose) plus a diluent such as lactose. The spheroids thus produced can be coated with a semi-permeable membrane (e.g. ethyl cellulose, Eudragit WE30D) to produce sustained release properties.

For infections of the eye or other external tissues, e.g., mouth and skin, the formulations are preferably applied as a topical ointment or cream containing the active ingredient in an amount of, for example, 0.075 to 20% w/w, preferably 0.2 to 15% w/w and most preferably 0.5 to 10% w/w. When formulated in an ointment, the active ingredients may be employed with either a paraffinic or a water-miscible ointment base. Alternatively, the active ingredients may be formulated in a cream with an oil-in-water cream base or as in a water in oil base.

If desired, the aqueous phase of the cream base may include, for example, at least 40-45% w/w of a polyhydric alcohol, i.e. an alcohol having two or more hydroxyl groups such as propylene glycol, butane-1,3-diol, mannitol, sorbitol, glycerol and polyethylene glycol and mixtures thereof. The topical formulations may desirably include a compound which enhances absorption or penetration of the active ingredient through the skin or other affected areas. Examples of such dermal penetration enhancers include dimethylsulphoxide and related analogues.

The oily phase of the emulsions of this invention may be constituted from known ingredients in a known manner. While this phase may comprise merely an emulsifier (otherwise known as an emulgent), it desirably comprises a mixture of at least one emulsifier with a fat or an oil or with both a fat and an oil. Preferably, a hydrophilic emulsifier is included together with a lipophilic emulsifier which acts as a stabiliser. It is also preferred to include both an oil and a fat. Together, the emulsifier(s) with or without stabiliser(s) make up the so-called emulsifying wax, and the wax together with the oil and/or fat make up the so-called emulsifying ointment base which forms the oily dispersed phase of the cream formulations.

Emulgents and emulsion stabilisers suitable for use in the formulation of the present invention include Tween 60, Span 80, cetostearyl alcohol, myristyl alcohol, glyceryl mono-stearate and sodium lauryl sulphate.

The compound (1S,4R)-cis-4-[2-amino-6-(cyclopropylamino)-9H-purin-9-yl]-2-cyclo-pentene-1-methanol can be synthesised in accordance with European Patent Specification Number 0434450 or alternatively PCT Application No. GB/9500225 which are incorporated herein by reference .

Succinic acid is commercially available (Aldrich Chemical Company, Dorset, England).

Example A

Preparation of (1S,4R)-cis-4-[2-amino-6-(cyclopropylamino)-9H-purin-9-yl]-2-cyclo-pentene-1-methanol Succinate Salt.

A solution of (1S,4R)-cis-4-[2-amino-6-(cyclopropylamino)-9H-purin-9-yl]-2-cyclopentene-1-methanol (30.93 g, 0.102 mole) in absolute ethanol (96 mL) was added to a solution of succinic acid (Aldrich, 99%, 12.15 g, 0.102 mole) in water (130mL). The mixture was brought to reflux and the resulting solution was treated with charcoal (0.4 g) and filtered through a Celite pad. Pale yellow crystals (38.0 g) formed as the solution cooled slowly to ambient temperature. This solid was recrystallized from water (500 mL) to give (1S,4R)-cis-4-[2-amino-6-(cyclopropylamino)-9H-purin-9-yl]-2-cyclopentene-1-methanol succinate as a white powder (34.9 g, 80%), m.p. 168-169°C; $^1$H-NMR (DMSO-$d_6$)δ: 12.70-11.70 (brm, 2, 2 carboxyl H), 7.62 (s, 1, purine CH), 7.30 (d, J = 4.1Hz), 6.10 (m, 1, =CH), 5.90- 5.75 (m, 3,=CH and NH$_2$), 5.40 (m, 1, NCH), 5.20-4.50 (br m, 1, OH), 3.46 (d, J = 5.9 Hz, 2, OCH$_2$), 3.10 (br m, 1, CH of cyclopropyl), 2.85 (br m, 1, CH), 2.70-2.50 (m, 1, CH), 2.40 (s, 4, 2 CH$_2$ of succinate), 1.70-1.50 (m, 1, CH), 0.75-0.50 (m, 4, 2 CH$_2$ of cyclopropyl).

Anal. Calcd. for C$_{14}$H$_{18}$N$_6$0.C$_4$H$_6$O$_4$.050 H$_2$O: C, 52.29 H, 6.09; N, 20.33. Found: C, 52.33; H, 6.09; N, 20.38.

Example B: Tablet Formulations

The following formulations A, B and C are prepared by wet granulation of the ingredients with a solution of povidone, followed by addition of magnesium stearate and compression.

Formulation A

|     |                          | mg/tablet | mg/tablet |
|-----|--------------------------|-----------|-----------|
| (a) | Active ingredient        | 250       | 250       |
| (b) | Lactose B.P.             | 210       | 26        |
| (c) | Povidone B.P.            | 15        | 9         |
| (d) | Sodium Starch Glycollate | 20        | 12        |
| (e) | Magnesium Stearate       | 5         | 3         |
|     |                          | 500       | 300       |

Formulation B

|     |                   | mg/tablet | mg/tablet |
|-----|-------------------|-----------|-----------|
| (a) | Active ingredient | 250       | 250       |

(continued)

|   |   | mg/tablet | mg/tablet |
|---|---|---|---|
| (b) | Lactose | 150 | - |
| (c) | Avicel PH 101 | 60 | 26 |
| (d) | Povidone B.P. | 15 | 9 |
| (e) | Sodium Starch Glycollate | 20 | 12 |
| (f) | Magnesium Stearate | 5 | 3 |
|   |   | 500 | 300 |

Formulation C (Controlled Release Formulation)

The formulation is prepared by wet granulation of the ingredients (below) with a solution of povidone followed by the addition of magnesium stearate and compression.

|   |   | mg/tablet |
|---|---|---|
| (a) | Active Ingredient | 500 |
| (b) | Hydroxypropylmethylcellulose (Methocel K4M Premium) | 112 |
| (c) | Lactose B.P. | 53 |
| (d) | Povidone B.P.C. | 28 |
| (e) | Magnesium Stearate | 7 |
|   |   | 700 |

Capsules are prepared by dispersing the active ingredient in the lecithin and arachis oil and filling the dispersion into soft, elastic gelatin capsules.

Formulation C (Controlled Release Capsule)

The following controlled release capsule formulation is prepared by extruding ingredients a, b, and c using an extruder, followed by spheronisation of the extrudate and drying. The dried pellets are then coated with release-controlling membrane (d) and filled into a two-piece, hard gelatin capsule.

|   |   | mg/capsule |
|---|---|---|
| (a) | Active Ingredient | 250 |
| (b) | Microcrystalline Cellulose | 125 |
| (c) | Lactose BP | 125 |
| (d) | Ethyl Cellulose | 13 |
|   |   | 513 |

Example D: Injectable Formulation

| Active ingredient | 0.200 g |
|---|---|
| Sterile, pyrogen free phosphate buffer (pH 7.0) to | 10 ml |

The active ingredient is dissolved in most of the phosphate buffer (35-40°C), then made up to volume and filtered through a sterile micropore filter into a sterile 10ml amber glass vial (type 1) and sealed with sterile closures and overseals.

Example E: Intramuscular Injection

| Active Ingredient | | 0.20 g |
|---|---|---|
| Benzyl Alcohol | | 0.10 g |
| Glycofurol 75 | | 1.45 g |
| Water for Injection | q.s. to | 3.00 ml |

The active ingredient is dissolved in the glycofurol. The benzyl alcohol is then added and dissolved, and water added to 3 ml. The mixture is then filtered through a sterile micropore filter and sealed in sterile 3 ml glass vials (type 1).

| Example F: | Syrup Suspension |
|---|---|
| Active ingredient | 0.2500 g |
| Sorbitol Solution | 1.5000 g |
| Glycerol | 2.0000 g |
| Dispersible Cellulose | 0.0750 g |
| Sodium Benzoate | 0.0050 g |
| Flavour, Peach 17.42.3169 | 0.0125 ml |
| Purified Water        q.s. to | 5.0000 ml |

The sodium benzoate is dissolved in a portion of the purified water and the sorbitol solution added. The active ingredient is added and dispersed. In the glycerol is dispersed the thickener (dispersible cellulose). The two dispersions are mixed and made up to the required volume with the purified water.

Example G: Suppository

| | mg/suppository |
|---|---|
| Active Ingredient (63lm) | 250 |
| Hard Fat, BP (Witepsol H15 - Dynamit NoBel) | 1770 |
| | 2020 |

*The active ingredient is used as a powder wherein at least 90% of the particles are of 63µm diameter or less.

One-fifth of the Witepsol H15 is melted in a steam-jacketed pan at 45°C maximum. The active ingredient is sifted through a 200µm sieve and added to the molten base with mixing, using a silverson fitted with a cutting head, until a smooth dispersion is achieved. Maintaining the mixture at 45°C, the remaining Witepsol H15 is added to the suspension and stirred to ensure a homogenous mix. The entire suspension is passed through a 250lm stainless steel screen and, with continuous stirring, is allowed to cool to 40°C. At a temperature of 38°C to 40°C 2.02g of the mixture is filled into suitable plastic moulds. The suppositories are allowed to cool to room temperature.

Example H: Pessaries

| | mg/pessary |
|---|---|
| Active ingredient 63lm | 250 |
| Anhydrous Dextrose | 380 |
| Potato Starch | 363 |
| Magnesium Stearate | 7 |
| | 1000 |

The above ingredients are mixed directly and pessaries prepared by direct compression of the resulting mixture.

Example I: Topical formulation

|  | Cream |
|---|---|
| Active compound | 5.00g |
| Glycerol | 2.00g |
| Cetostearyl alcohol | 6.75g |
| Sodium lauryl sulphate | 0.75g |
| White soft paraffin | 2.50g |
| Liquid paraffin | 5.00g |
| Chlorocresol | 0.10g |
| Purified water        to | 100.00g |

Dissolve the active compound in a mixture of purified water and glycerol and heat to 70°C. Heat the remaining ingredients together at 70°C. Add the two parts together and emulsify. Cool and fill into containers.

**Claims**

1. The succinate salt of (1S,4R)-cis-4-[2-amino-6-(cyclopropylamino)-9H-purin-9-yl]-2-cyclopentene-1-methanol.

2. A solvate of the compound as claimed in Claim 1.

3. A hydrate of the compound as claimed in Claim 1.

4. A compound as claimed in any Claim from 1 to 3 for use in medical therapy.

5. Use of a compound as claimed in any claim from 1 to 3 in the manufacture of a medicament for the treatment or prophylaxis of a viral infection.

6. A process for the preparation of the compound claimed in Claim 1 which comprises mixing (1S,4R)-cis-4-[2-amino-6-(cyclopropylamino)-9H-purin-9-yl]-2-cyclopentene-1-methanol in a suitable solvent with succinic acid in water and at such conditions so as to effect the formation of the compound of Claim 1.

7. A pharmaceutical formulation comprising a compound claimed in any Claim from 1 to 3 and at least one pharmaceutically acceptable carrier thereto.

8. A pharmaceutical formulation as claimed in Claim 7 formed into a tablet or a capsule.

9. A pharmaceutical formulation as claimed in Claim 7 adapted for parenteral administration.

**Patentansprüche**

1. Succinatsalz von (1S,4R)-cis-4-[2-Amino-6-(cyclopropylamino)-9H-purin-9-yl]-2-cyclopenten-1-methanol.

2. Solvat der Verbindung gemäß Anspruch 1.

3. Hydrat der Verbindung gemäß Anspruch 1.

4. Verbindung gemäß einem der Ansprüche 1 bis 3 zur Verwendung in der medizinischen Therapie.

**5.** Verwendung einer Verbindung gemäß einem der Ansprüche 1 bis 3 in der Herstellung eines Medikaments zur Behandlung oder Prophylaxe einer Virusinfektion.

**6.** Verfahren zur Herstellung der Verbindung gemäß Anspruch 1, welches das Vermischen von (1S,4R)-cis-4-[2-Amino-6-(cyclopropylamino)-9H-purin-9-yl]-2-cyclopenten-1-methanol in einem geeigneten Lösungsmittel mit Bernsteinsäure in Wasser und bei solchen Bedingungen umfaßt, um die Bildung der Verbindung des Anspruchs 1 zu bewirken.

**7.** Pharmazeutische Formulierung, die eine Verbindung gemäß einem der Ansprüche 1 bis 3 und wenigstens einen pharmazeutisch akzeptablen Träger dafür umfaßt.

**8.** Pharmazeutische Formulierung gemäß Anspruch 7, die zu einer Tablette oder Kapsel geformt ist.

**9.** Pharmazeutische Formulierung gemäß Anspruch 7, die zur parenteralen Verabreichung angepaßt ist.


**Revendications**

**1.** Sel succinate du (1S,4R)-cis-4-[2-amino-6-(cyclopropylamino)-9H-purin-9-yl]-2-cyclopentène-1-méthanol.

**2.** Solvate du composé selon la revendication 1.

**3.** Hydrate du composé selon la revendication 1.

**4.** Composé selon l'une quelconque des revendications 1 à 3, à utiliser en thérapie médicale.

**5.** Utilisation d'un composé selon l'une quelconque des revendications 1 à 3 dans la préparation d'un médicament pour le traitement ou la prophylaxie d'une infection virale.

**6.** Procédé pour la préparation du composé selon la revendication 1, qui comprend le fait de mélanger du (1S,4R)-cis-4-[2-amino-6-(cyclopropylamino)-9H-purin-9-yl]-2-cyclopentène-1-méthanol dans un solvant approprié avec de l'acide succinique dans de l'eau et dans des conditions telles que l'on obtient la formation du composé selon la revendication 1.

**7.** Formulation pharmaceutique comprenant un composé selon l'une quelconque des revendications 1 à 3 et au moins un support pharmaceutiquement acceptable pour ledit composé.

**8.** Formulation pharmaceutique selon la revendication 7, façonnée en comprimé ou en capsule.

**9.** Formulation pharmaceutique selon la revendication 7, conçue pour une administration par voie parentérale.